(19) Europäisches Patentamt
European Patent Office
Office européen des brevets

(11) EP 4 117 637 B1

(12) **EUROPEAN PATENT SPECIFICATION**

(45) Date of publication and mention of the grant of the patent:
**16.09.2026 Bulletin 2026/38**

(21) Application number: **21713779.3**

(22) Date of filing: **12.03.2021**

(51) International Patent Classification (IPC):
***A61K 9/16*** *(2006.01)* ***A61K 9/20*** *(2006.01)*
***A61K 31/4439*** *(2006.01)*

(52) Cooperative Patent Classification (CPC):
**A61K 9/1688; A61K 9/2013; A61K 9/2018; A61K 9/2059; A61K 31/4439**

(86) International application number:
**PCT/PT2021/050006**

(87) International publication number:
**WO 2021/182981 (16.09.2021 Gazette 2021/37)**

(54) **MICRONISED OPICAPONE**

MIKRONISIERTES OPICAPON

OPICAPONE MICRONISÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **13.03.2020 GB 202003705**
**26.05.2020 GB 202007814**

(43) Date of publication of application:
**18.01.2023 Bulletin 2023/03**

(73) Proprietor: **Bial-Portela & CA, S.A.**
**4745-457 S. Mamede do Coronado (PT)**

(72) Inventors:
- **VASCONCELOS, Teófilo**
**4745-457 S. Mamede do Coronado (PT)**
- **RUSSO, Domenico**
**4745-457 S. Mamede do Coronado (PT)**
- **GONÇALVES DA CRUZ RAMOS PIRES, Ana Rita**
**4745-457 S. Mamede do Coronado (PT)**
- **CORREIA DIAS, Sonia Maria**
**4745-457 S. Mamede do Coronado (PT)**

(74) Representative: **Elkington and Fife LLP**
**Prospect House**
**8 Pembroke Road**
**Sevenoaks, Kent TN13 1XR (GB)**

(56) References cited:
**WO-A1-2013/089573**

Remarks:
The file contains technical information submitted after the application was filed and not included in this specification

EP 4 117 637 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of Invention

This invention relates to micronised pharmaceutical products consisting essentially of crystalline opicapone. The invention also relates to a method of producing these micronised pharmaceutical products and their use in improving the bioavailability of opicapone in the treatment of Parkinson's disease. Furthermore, the invention relates to methods for determining the primary particle size distribution and the agglomerate content within such micronised pharmaceutical products.

### Background to the Invention

Levodopa (L-DOPA) has been used in clinical practice for several decades in the symptomatic treatment of various conditions, including Parkinson's disease. L-DOPA is able to cross the blood-brain barrier, where it is then converted to dopamine and increases the levels thereof. However, conversion of L-DOPA to dopamine may also occur in the peripheral tissue, possibly causing adverse effects upon administration of L-DOPA. Therefore, it has become standard clinical practice to co-administer a peripheral amino acid decarboxylase (AADC) inhibitor, such as carbidopa or benserazide, which prevents conversion to dopamine in peripheral tissue. It is also known that inhibitors of the enzyme catechol-O-methyltransferase (COMT) may provide clinical improvements in patients afflicted with Parkinson's disease undergoing treatment with L-DOPA, since COMT catalyses the degradation of L-DOPA.

It has been found, as set forth in International Publication No. WO 2007/013830, that the nitrocatechol derivative opicapone is a potent and long-acting COMT inhibitor. This compound is bioactive, bioavailable and exhibits low toxicity. Thus, opicapone has potentially valuable pharmaceutical properties in the treatment of some central and peripheral nervous system disorders where inhibition of O-methylation of catecholamines may be of therapeutic benefit, such as, for example, mood disorders; movement disorders, such as Parkinson's disease, parkinsonian disorders and restless legs syndrome; gastrointestinal disturbances; oedema formation states; and hypertension. The development of the opicapone molecule is described in L. E. Kiss et al, J. Med. Chem., 2010, 53, 3396-3411 and it was approved for marketing in the EU in June 2016.

Further research since WO 2007/013830 has focused on optimising opicapone into a stable and bioavailable form. For example, WO 2009/116882 describes various polymorphs of opicapone, with polymorph A being both kinetically and thermodynamically stable. WO 2010/114404 and WO 2010/114405 describe stable opicapone formulations used in clinical trials. WO 2013/089573 describes optimised methods for producing opicapone using simple starting materials and with good yields. Importantly, WO 2013/089573 also discloses that when recrystallised opicapone is ball milled or micronized through spiral jet mills, microparticles of the desired size for good oral bioavailability can be obtained. This effect is supported by the poster abstract "Relative Bioavailability of Opicapone from Two Different Formulations in Healthy Subjects: The In Vivo Effect of Particle Size" (R. Lima et al, AAPS Annual Meeting, Orlando, 2015), which describes a phase I clinical trial in healthy volunteers comparing the bioavailability ($AUC_{0\text{-}inf}$ and $C_{max}$) of micronised and non-micronised opicapone. WO 2013/089573 discloses Equivalent Circular Diameter (ECD) values (D10, D50 and D95) characteristic of micronised opicapone with bioavailability ~2-fold higher than the non-micronised equivalent. Therefore, the preferred opicapone form for clinical use is based on a pharmaceutical product consisting essentially of crystalline opicapone substance with the ECD size characteristics described in WO 2013/089573.

In spite of being consistently more bioavailable than the non-micronised form, the inventors have since discovered that final drug product formulations containing micronised crystalline opicapone may still vary considerably in their oral bioavailability (e.g. AUC and $C_{max}$). This variability was observed in spite of the pharmaceutical product being produced according to good manufacturing practices and fulfilling the ECD size characteristics described in WO 2013/089573.

Therefore, there remains a need for a pharmaceutical product consisting essentially of crystalline opicapone that can be formulated together with suitable pharmaceutical excipients to provide a final drug product which has improved oral bioavailability and consistent pharmacokinetic parameters (e.g. AUC and $C_{max}$) so as to ensure bioequivalence in humans and/or animal models. Additionally, there remains a need for methods of characterising a pharmaceutical product consisting essentially of crystalline opicapone that can predict whether the pharmaceutical product can be formulated together with suitable pharmaceutical excipients to provide a final drug product which has improved oral bioavailability and consistent pharmacokinetic parameters (e.g. AUC and $C_{max}$) so as to ensure bioequivalence in humans and/or animal models.

### Summary of the Invention

The present inventors have now identified a previously unknown characteristic of micronised pharmaceutical

products consisting essentially of crystalline opicapone which can cause biologically significant batch-to-batch variability in pharmacokinetic parameters (e.g. AUC and $C_{max}$) in spite of displaying comparable primary particle size distribution, as characterised using the standard ECD values (D10, D50 and/or D95) described in WO 2013/089573.

The inventors discovered that the bioavailability of such products could be improved and biologically significant batch-to-batch variability eliminated when the agglomerate distribution of micronised crystalline opicapone was analysed and the proportion of sheaf agglomerates was low (≤ 30%) and, preferably, the proportion of globular aggregates was high (≥ 70%). In batches where these criteria were not met, repeat micronisation, preferably by jet milling, as described below, resulted it a micronised product fulfilling these criteria.

Accordingly, in a first general embodiment, the invention provides a pharmaceutical product consisting essentially of crystalline opicapone having the following primary particle size distribution:

D10 (maximum distance) greater than or equal to 8 μm;
D50 (maximum distance) of 20 to 55 μm; and
D90 (maximum distance) less than or equal to 200 μm;

and the following agglomerate distribution:

% number of sheaf agglomerates less than or equal to 25%;
wherein crystalline opicapone comprises at least 95 wt% of the total dry weight of the pharmaceutical product;
wherein the term "sheaf agglomerate" is defined as measured in Experiment 1 of the description;

wherein the "maximum distance" and "primary particle size distribution" are measured as described in Experiment 2 of the description; and
wherein the term "agglomerate" refers to an assemblage of at least 10 primary particles of crystalline opicapone.

In a second general embodiment, the invention provides a further pharmaceutical product comprising the pharmaceutical product according to the first general embodiment blended with one or more pharmaceutically acceptable excipients.

In a third general embodiment, the invention provides a further pharmaceutical product wherein the pharmaceutical product according to the second general embodiment is granulated.

In a fourth general embodiment, the invention provides a further pharmaceutical product comprising the pharmaceutical product according to the third general embodiment blended with one or more pharmaceutically acceptable excipients.

In a fifth general embodiment, the invention provides a capsule for oral administration comprising a pharmaceutical product according to any one of the second, third or fourth general embodiments.

In a sixth general embodiment, the invention provides a tablet for oral administration comprising a pharmaceutical product according to any one of the second, third or fourth general embodiments.

In a seventh general embodiment, the invention provides method of manufacturing a pharmaceutical product comprising the following steps:

a) micronising a product consisting essentially of crystalline opicapone;
b) determining the primary particle size distribution and the % number of sheaf agglomerates for the crystalline opicapone in the micronized product;
c) retaining micronized product consisting essentially of crystalline opicapone having the following primary particle size distribution:

D10 (maximum distance) greater than or equal to 8 μm;
D50 (maximum distance) of 20 to 55 μm; and
D90 (maximum distance) less than or equal to 200 μm;

and the following agglomerate distribution:
% number of sheaf agglomerates less than or equal to 25%; and
d) if necessary, repeating steps a) to c) on micronized product consisting essentially of crystalline opicapone which does not have the primary particle size and agglomerate distributions defined in step c) above;

wherein crystalline opicapone comprises at least 95 wt% of the total dry weight of the pharmaceutical product;

wherein the term "sheaf agglomerate" is defined as measured in Experiment 1 of the description;

wherein the "maximum distance" and "primary particle size distribution" are measured as described in Experiment 2 of the description; and
wherein the term "agglomerate" refers to an assemblage of at least 10 primary particles of crystalline opicapone.

In an eight general embodiment, the invention provides a medicament comprising a pharmaceutical product as defined in the first general embodiment, for use in increasing opicapone bioavailability in a patient suffering from Parkinson's disease, as compared to the opicapone bioavailability which would be obtained from an equivalent medicament comprising a pharmaceutical product as defined in the first general embodiment except for having a percentage number of sheaf agglomerates greater than 30%.

Further specific and preferred aspects of these general embodiments are described below.

## Brief Description of the Figures

**Figure 1** shows pictures of a typical **"sheaf agglomerate"** sitting amongst disaggregated primary particles of crystalline opicapone.
**Figure 2** shows pictures of a typical **"globular agglomerate"** sitting amongst disaggregated primary particles of crystalline opicapone.
**Figure 3** shows preferred aspect ratio and solidity values for globular agglomerates.
**Figure 4** shows the **"equivalent circle diameter"** (ECD) of a particle (a).
**Figure 5** shows the **"maximum distance"** of a particle (b).
**Figure 6** shows the **"total fibre length"** of a fibrous particle (c).
**Figure 7** shows the correlation between **"total fibre length"** and **"maximum distance"** of a particle.
**Figure 8** shows the correlation between sheaf agglomerates and globular agglomerates.
**Figure 9** shows plasma levels of opicapone following a single oral administration of various micronized crystalline opicapone samples to male Wistar rats (see Experiment 4.1 below).

## Detailed Description of the Invention

### A. Definitions

The following definitions apply to the terms as used throughout this specification, unless otherwise limited in specific instances.

A **"pharmaceutical product"** is a product which can be used to prepare a final medicament or drug product suitable for administration to a patient.

The term **"consisting essentially of crystalline opicapone"** means that the pharmaceutical product consists entirely of crystalline opicapone, or it consists of crystalline opicapone with only small amounts of other components which do not materially affect its essential pharmaceutical properties. The pharmaceutical product consisting essentially of crystalline opicapone contains crystalline opicapone in an amount of at least 95 wt%, preferably at least 97 wt%, more preferably at least 98 wt%, even more preferably at least 99 wt%, based on the total dry weight of the pharmaceutical product.

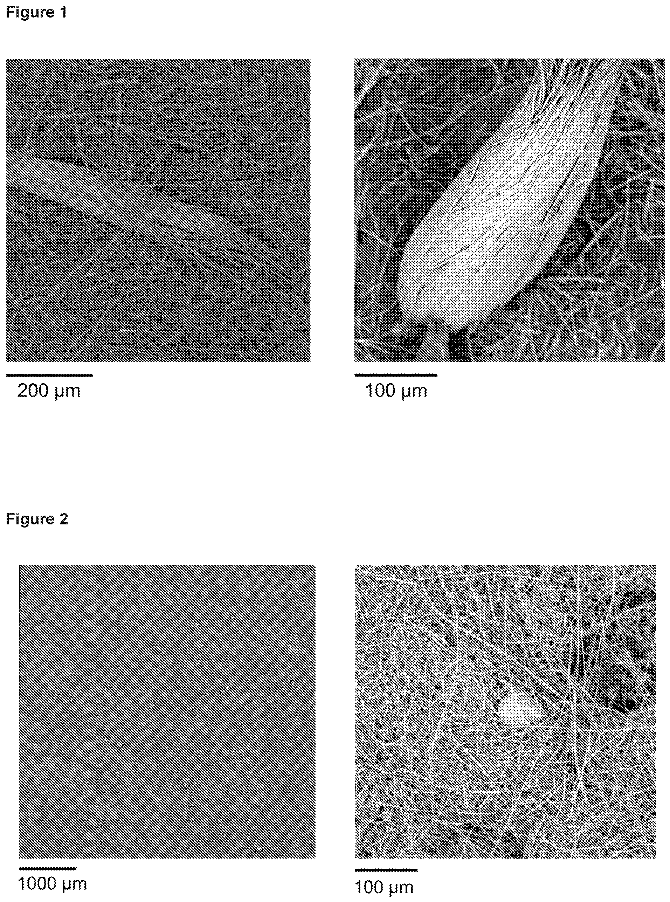

The term **"primary particles"** refers to the smallest discrete identifiable crystalline opicapone entities within a sample of the pharmaceutical product. A primary particle may consist of a single crystal of opicapone. As can be seen from **Figures 1 and 2,** primary particles of crystalline opicapone are typically rod-shaped and/or needle-shaped and/or fibrous.

An **"agglomerate"** of crystalline opicapone refers to an assemblage of at least 10 primary particles of crystalline opicapone, usually held together by weak physical interactions. Typically, such agglomerates contain many more primary particles of crystalline opicapone. The formation of agglomerates is generally reversible and an agglomerate can usually be converted to discrete primary particles by application of a relatively weak force.

A **"sheaf agglomerate"** of crystalline opicapone is an agglomerate wherein the primary particles are predominantly assembled side-by-side. Such agglomerates are assembled in a manner that may, for example, resemble a corn sheaf (see **Figure 1).** Typically, such agglomerates have at least 60%, more typically at least 70%, still more typically at least 80% of their primary particles assembled side-by-side. Unlike most agglomerates, a sheaf agglomerate of crystalline opicapone is not easily converted (e.g. disaggregated) to discrete primary particles. A **"sheaf agglomerate"** may be further defined as having an **'aspect ratio'** less than 0.45 (or, as defined in the claims, an **'elongation'** greater than 0.55, since elongation = 1 - aspect ratio). The **'aspect ratio'** is equal to the **'width'** of the agglomerate divided by its **'length',** wherein the **'length'** is calculated by projecting all possible lines from one point on the perimeter of the agglomerate to another point on its perimeter onto the **'major axis'** (the **'major axis'** being the axis of minimum rotational

energy) and measuring the maximum length of these projections, and the **'width'** is calculated by projecting all possible lines from one point on the perimeter of the agglomerate to another point on its perimeter onto the **'minor axis'** (the **'minor axis'** being the axis of maximum rotational energy) and measuring the maximum length of these projections.

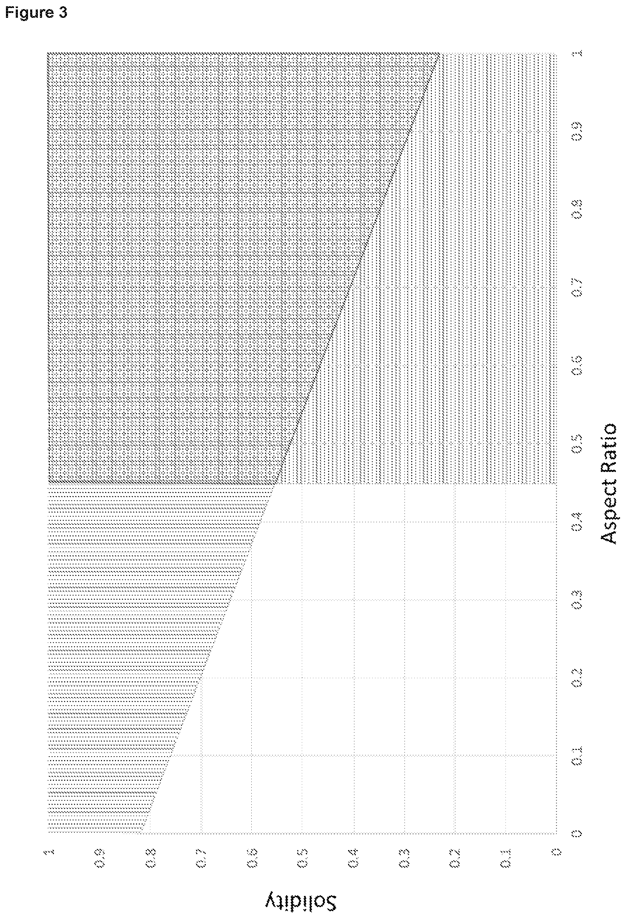

A **"globular agglomerate"** of crystalline opicapone is an agglomerate wherein the primary particles are arranged in a manner other than as a "sheaf agglomerate". Usually, this results in a substantially spherical or globe-like agglomerate (see **Figure 2).** Like most agglomerates, a globular agglomerate of crystalline opicapone is easily converted to discrete primary particles. A **"globular agglomerate"** may be further defined as having an **'aspect** ratio' greater than or equal to 0.45 (or, as defined in the claims, an **'elongation'** less than or equal to 0.55, since elongation = 1 - aspect ratio). A **"globular agglomerate"** may be still further defined as a **"polygon"** having [solidity : aspect ratio] coordinates within the region of a solidity (y-axis) versus aspect ratio (x axis) graph defined by the vertices [0.23:1], [0.82:0], [1:0] and [1;1]. The **'aspect ratio'** is as defined above and the **'solidity'** is equal to the area bound by the actual perimeter of the agglomerate divided by the area bound by its **'convex hull perimeter'.** The **'convex hull perimeter'** is a well-established parameter which, in simple terms, may be envisaged as an imaginary elastic band stretched around the outline of the particle image. Thus, a polygon having an aspect ratio of 1, may have a wide range of solidity (i.e. 0.23 to 1) whereas a polygon having an aspect ratio tending towards 0, must lie within a narrow range for solidity (i.e. 0.82 to 1). Of course, a globular agglomerate preferably has an aspect ratio greater than or equal to 0.45. Therefore, agglomerates meeting the polygon criteria do not necessarily qualify as preferred globular agglomerates; and globular agglomerates which meet the aspect ratio criterion do not necessarily qualify as polygons. However, particularly preferred globular agglomerates meet both the aspect ratio criterion and also the polygon criterion (see cross-hatched region of **Figure 3).**

Globular agglomerates generally require less energy than sheaf agglomerates to convert them into discrete primary particles. In other words, a stronger force is generally required to break up a sheaf agglomerate than a globular agglomerate.

The term **"% number of sheaf agglomerates"** refers to the number of sheaf agglomerates in the pharmaceutical product expressed as a percentage of the total number of all types of agglomerate present in the pharmaceutical product. Similarly, the term "% **number of globular agglomerates"** refers to the number of globular agglomerates in the pharmaceutical product expressed as a percentage of the total number of all types of agglomerate present in the pharmaceutical product.

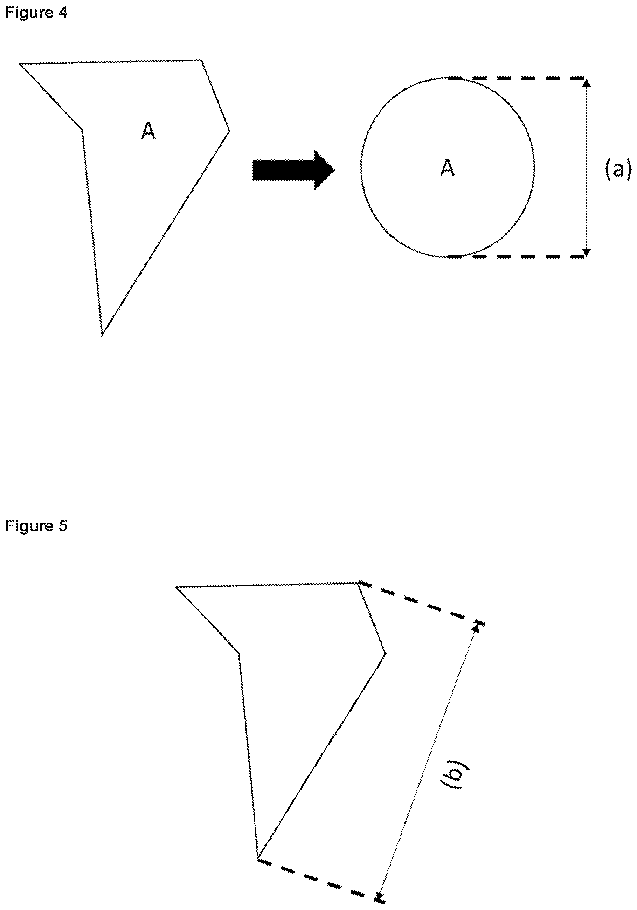

The **"equivalent circle diameter"** (ECD) of a particle is the diameter of a circle with the same area A as the projected area of the particle image (see **Figure 4).**

The **"maximum distance"** of a particle, as defined in the claims, is the furthest distance between any two points of the particle (see **Figure 5),** preferably as visualised using light microscopy or scanning electron microscopy.

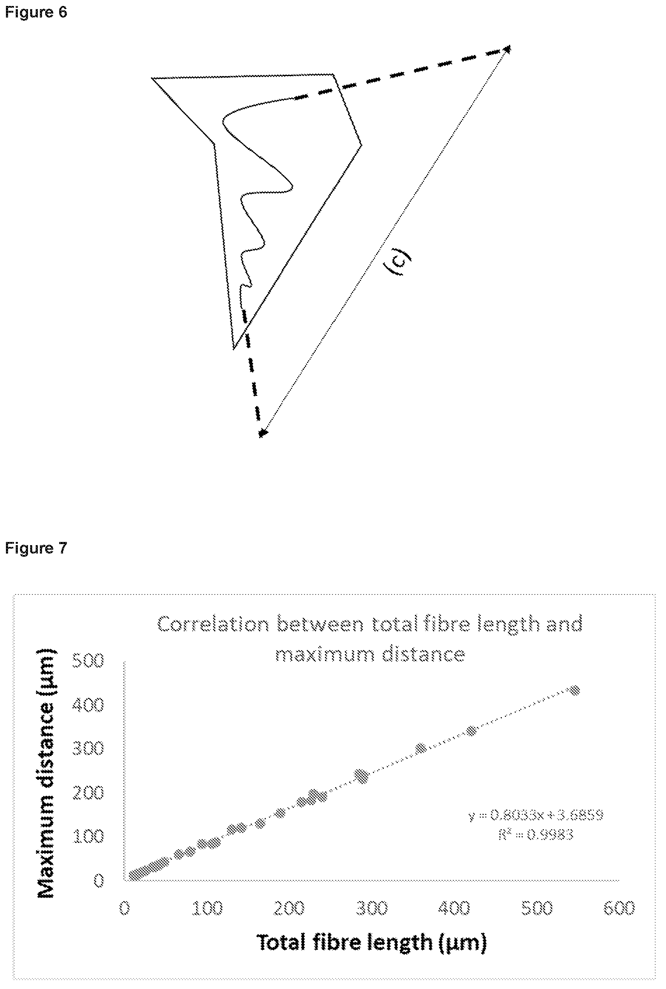

The **"total fibre length"** refers to the length of a fibrous particle as if it was straightened out. It can be assessed by analysis of the skeleton of the fibre and subsequent derivation of its length, also including the particle's branches (if any are present) (see **Figure 6).**

During the investigations which led to the present invention, the inventors measured both the maximum distance and total fibre length for different batches of pharmaceutical product consisting essentially of crystalline opicapone and surprisingly found that these parameters correlate directly in a predictable manner (see **Figure 7).** Due to the fact that maximum distance is quicker to measure and computationally less expensive, this parameter is preferred. However, it is within the scope of the invention to measure alternative parameters of particle size that correlate with maximum distance in a predictable manner. For example, for a micronised pharmaceutical product consisting essentially of crystalline opicapone, total fibre length can be measured instead and approximately converted into maximum distance by multiplying the total fibre length by 0.8. To ensure conversion between equivalent parameters is predictable, a correlation factor ($R^2$) of at least 0.90 preferably 0.95, is required.

Other variations to the disclosed embodiments can be understood and effected by those skilled in the art in practicing the claimed invention, from a study of the disclosure, and the appended claims. In the claims, the word **"comprising"** does not exclude other elements or steps, and the indefinite article "a" or "an" does not exclude a plurality. The mere fact that certain measures are recited in mutually different dependent claims does not indicate that a combination of these measures cannot be used to advantage.

## B. Pharmaceutical products

The invention provides a pharmaceutical product consisting essentially of crystalline opicapone having a specific primary particle size distribution and a percentage number of sheaf agglomerates less than or equal to 25%.

The inventors surprisingly discovered that a pharmaceutical product with these characteristics could be used to prepare a final medicament or drug product suitable for administration to a patient which displayed good oral bioavailability (e.g. AUC and $C_{max}$) whilst batch-to-batch variability was reduced. In particular, pharmaceutical products with these characteristics did not result in batches which, when formulated into a final medicament or drug product, suffered a significant reduction in bioavailability. In this respect, a **"significant reduction in bioavailability"** is defined as a

reduction in a particular pharmacokinetic parameter (e.g. AUC and/or $C_{max}$) such that the final medicament or drug product may no longer be considered bioequivalent to that approved by the relevant regulatory authorities. The term **"bioequivalent"** is known to the skilled person and generally refers to a final medicament or drug product having a bioavailability (e.g. AUC and $C_{max}$) in the range of 80 to 125% of standard parameters established for the final medicament or drug product as approved by the relevant regulatory authorities.

Generally, the micronised pharmaceutical product consisting essentially of crystalline opicapone has the following primary particle size distribution:

i) D10 (maximum distance) greater than or equal to 8 μm;
ii) D50 (maximum distance) of 20 to 55 μm; and
iii) D90 (maximum distance) less than or equal to 200 μm;

Therefore, the pharmaceutical product consists essentially of crystalline opicapone having the following primary particle size and agglomerate distributions:

i) D10 (maximum distance) greater than or equal to 8 μm;
ii) D50 (maximum distance) of 20 to 55 μm;
iii) D90 (maximum distance) less than or equal to 200 μm; and
iv) % number of sheaf agglomerates less than or equal to 25%.

The crystalline opicapone of the micronised pharmaceutical product has a percentage number of sheaf agglomerates less than or equal to 25%, more preferably less than or equal to 20%, even more preferably less than or equal to 15% and most preferably less than or equal to 10%. These lower levels of sheaf agglomerates may provide enhanced bioavailability (e.g. AUC and $C_{max}$), for example, over a product with more than 30% of sheaf agglomerates.

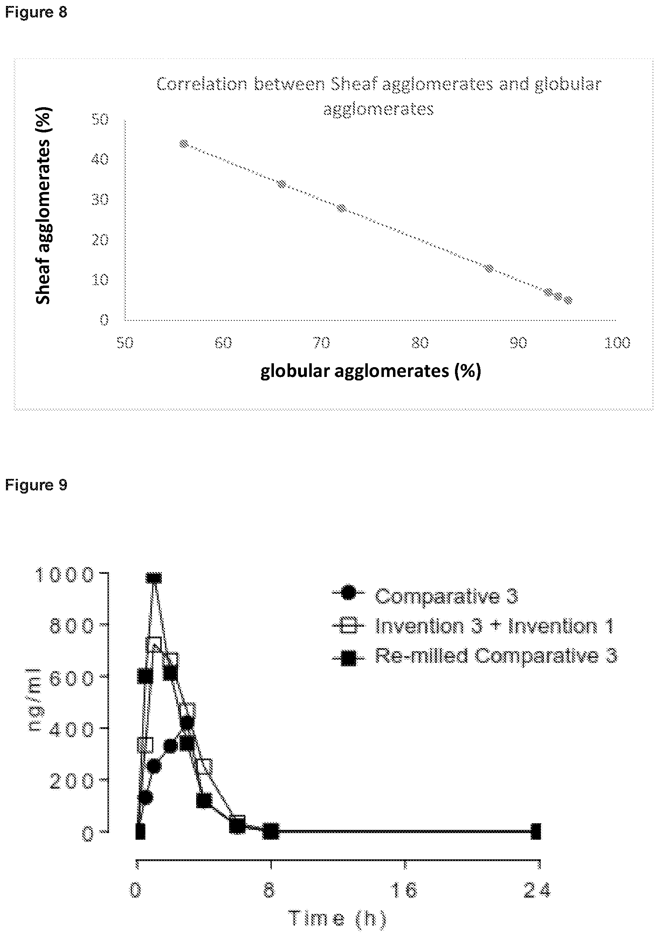

Alternatively, or additionally, increased bioavailability (e.g. AUC and $C_{max}$) and reduced batch-to-batch variability can be predicted based upon a high level of globular agglomerates within the pharmaceutical product. This is because the inventors discovered that the agglomerates in the crystalline opicapone of the micronised pharmaceutical product mainly consist of sheaf and globular agglomerates (see **Figure 8).** As such, a percentage number of globular agglomerates more than or equal to 75% is equivalent a percentage number of sheaf agglomerates less than or equal to 25%. The crystalline opicapone of the micronised pharmaceutical product has a percentage number of globular agglomerates more than or equal to 75%, more preferably more than or equal to 80%, even more preferably more than or equal to 85% and most preferably more than or equal to 90%.

In a preferred embodiment, the total area occupied by sheaf agglomerates in a 1 mg sample of the pharmaceutical product, as determined by particle size measurement (such as that described in Experiment 1 below), is lower than $4.0 \times 10^6$ $\mu m^2$/mg, more preferably lower than $3.0 \times 10^6$ $\mu m^2$/mg, even more preferably lower than $2.0 \times 10^6$ $\mu m^2$/mg, most preferably lower than $1.0 \times 10^6$ $\mu m^2$/mg.

In a preferred embodiment, the total volume occupied by sheaf agglomerates in a 1 mg sample of the pharmaceutical product, as determined by particle size measurement (such as that described in Experiment 1 below), is lower than $5 \times 10^8$ $\mu m^3$/mg, more preferably lower than $3.0 \times 10^8$ $\mu m^3$/mg, even more preferably lower than $2.0 \times 10^8$ $\mu m^3$/mg, most preferably lower than $1.0 \times 10^8$ $\mu m^3$/mg.

In an even more preferred embodiment, the crystalline opicapone has the following primary particle size distribution:

i) D10 (maximum distance) greater than or equal to 9 μm;
ii) D50 (maximum distance) of 25 to 50 μm; and/or
iii) D90 (maximum distance) less than or equal to 180 μm.

These values are particularly suitable and displayed optimal bioavailability with bioequivalence observed provided that large amounts of sheaf agglomerates (i.e. more than 30%) are not present.

The pharmaceutical product of the invention consists essentially of micronised crystalline opicapone. This is because pharmaceutical products with large amounts of impurities and/or other pharmaceutical ingredients (e.g. pharmaceutical excipients) are not amenable to the processes of determining the primary particle size distribution, total fibre length distribution and/or agglomerate distribution of the pharmaceutical product, described below. It would not be possible to accurately distinguish primary particles and/or agglomerates of micronised crystalline opicapone from other particles present. For example, a final medicament or drug product with 25 to 50 mg of opicapone will have been combined with relatively large amounts of pharmaceutical excipients and cannot be analysed using the methods described below. Therefore, the pharmaceutical product comprises crystalline opicapone in an amount of at least 95 wt%, preferably at least 97 wt%, more preferably at least 98 wt%, even more preferably at least 99 wt%, of the total dry weight of the pharmaceutical

product. Such purity levels make the pharmaceutical product particularly suitable for characterisation by the methods described below.

In another preferred embodiment, the crystalline opicapone of the pharmaceutical product is polymorph A disclosed in WO2009/116882. This polymorph displays excellent kinetic and thermodynamic stability, excellent bioavailability and is particularly suitable for micronisation processes described for opicapone.

**C. Methods of manufacture**

Methods for the synthesis, purification, crystallisation and micronisation of opicapone are known to those skilled in the art, and are described in the background section. However, the present invention also provides a method of manufacturing the pharmaceutical product described above comprising the following steps:

a) micronising a product consisting essentially of crystalline opicapone;
b) determining the primary particle size distribution and the % number of sheaf agglomerates in the micronized product;
c) retaining micronized product consisting essentially of crystalline opicapone having the following primary particle size distribution:

D10 (maximum distance) greater than or equal to 8 μm;
D50 (maximum distance) of 20 to 55 μm; and
D90 (maximum distance) less than or equal to 200 μm;

and the following agglomerate distribution:
% number of sheaf agglomerates less than or equal to 25%; and
d) if necessary, repeating steps a) to c) on micronized product consisting essentially of crystalline opicapone which does not have the primary particle size and agglomerate distributions defined in step c) above;

wherein crystalline opicapone comprises at least 95 wt% of the total dry weight of the pharmaceutical product;

wherein the term "sheaf agglomerate" is defined as measured in Experiment 1 of the description;
wherein the "maximum distance" and "primary particle size distribution" are measured as described in Experiment 2 of the description; and
wherein the term "agglomerate" refers to an assemblage of at least 10 primary particles of crystalline opicapone.

The claimed method allows a person skilled in the art to (1) identify batches of pharmaceutical product with appropriate bioavailability and reduced batch-to-batch variability, and (2) establish micronisation conditions that are highly suitable to convert batches of micronised opicapone with excessive percentage numbers of sheaf agglomerates into a pharmaceutical product according to the invention.

The inventors discovered that the following micronisation methods were most suitable for reducing the level of sheaf agglomerates. Preferably, the micronisation is performed by milling (and/or re-milling) using a jet-milling process with feed rates between 100 and 400 g/30 sec and milling pressures between 2.0 and 7.0 bar.

In instances where it is suspected or known that large amounts of sheaf agglomerates are present in a batch of micronised crystalline opicapone, the application also provides a method of manufacturing a pharmaceutical product comprising the following steps:

a) jet milling a micronised product consisting essentially of crystalline opicapone having, or suspected of having, a % number of sheaf agglomerates greater than 30%;
b) determining the primary particle size distribution and the % number of sheaf agglomerates for the crystalline opicapone in the micronized product;
c) retaining micronized product consisting essentially of crystalline opicapone having the following primary particle size distribution:

D10 (maximum distance) greater than or equal to 8 μm;
D50 (maximum distance) of 20 to 55 μm; and
D90 (maximum distance) less than or equal to 200 μm;

and the following agglomerate distribution:
% number of sheaf agglomerates less than or equal to 25%; and

d) if necessary, repeating steps a) to c) on micronized product consisting essentially of crystalline opicapone which does not have the primary particle size and agglomerate distributions defined in step c) above;

wherein crystalline opicapone comprises at least 95 wt% of the total dry weight of the pharmaceutical product;

wherein the term "sheaf agglomerate" is defined as measured in Experiment 1 of the description;
wherein the "maximum distance" and "primary particle size distribution" are measured as described in Experiment 2 of the description; and
wherein the term "agglomerate" refers to an assemblage of at least 10 primary particles of crystalline opicapone.

A micronised product would be known to contain this level of sheaf agglomerates if it had been analysed using the process described below. A micronised product would be suspected of containing this level of sheaf agglomerates if it has been manufactured using the same process as a batch of micronised product known to contain this level of sheaf agglomerates.

Once it has been established that pharmaceutical product is in accordance with the invention, it can be further processed into a final medicament or drug product safe in the knowledge that bioequivalence will be achieved. Therefore, in a generally preferred embodiment, the micronised pharmaceutical product retained in step c) of the method described above is combined with one or more pharmaceutically acceptable excipients to form a pharmaceutical composition (e.g. a medicament or drug product) suitable for oral administration. Accordingly, a preferred embodiment of the invention is directed to methods of manufacturing a pharmaceutical composition comprising (i) a therapeutically effective amount of the pharmaceutical product as defined above (e.g. an amount which provides 25 to 50 mg of opicapone); and (ii) one or more pharmaceutically acceptable excipients.

Preferably, the method involves the formation of granules of the pharmaceutical product and the one or more excipients. More preferably, the method involves formation of a unit dose of the granules. Even more preferably, the unit dose is a capsule or a tablet.

The pharmaceutical product manufactured according to the method of the invention may be administered alone or in combination with one or more other drugs (for example, a dopamine precursor and/or an AADC inhibitor). Generally, the dopamine precursor and/or AADC inhibitor will be administered as a single formulation in association with one or more pharmaceutically acceptable excipients and will be administered at least 1 hour before or after the pharmaceutical composition manufactured according to the method of the invention.

Pharmaceutical compositions suitable for the delivery of compounds of the present invention and methods for their preparation will be readily apparent to those skilled in the art. Such compositions and methods for their preparation may be found, for example, in "Remington's Pharmaceutical Sciences", 19th Edition (Mack Publishing Company, 1995). Particularly suitable excipients include lactose monohydrate, sodium starch glycolate, pregelatinized maize starch and magnesium stearate. Particularly suitable dosage forms for the pharmaceutical composition include capsules and tablets.

The method is particularly suitable for use in manufacturing pharmaceutical products and pharmaceutical formulations comprising pharmaceutical products with any or all of the preferred features described above in Section B, above.

**D. Methods of use**

This invention is also directed in part to a medicament comprising a pharmaceutical product of the invention, for use in increasing opicapone bioavailability in a patient suffering from Parkinson's disease, as compared to the opicapone bioavailability which would be obtained from an equivalent medicament comprising a pharmaceutical product of the invention except for having a percentage number of sheaf agglomerates greater than 30%.

In a preferred aspect of the invention, the use described above increases a relevant parameter of opicapone bioavailability (e.g. AUC and/or $C_{max}$) by at least 20%. The increase in bioavailability is compared to the opicapone bioavailability which would be obtained from an equivalent medicament manufactured using a pharmaceutical product of the invention except for having a percentage number of sheaf agglomerates greater than 30%.

In another preferred aspect of the invention, the opicapone is co-administered to the patient suffering from Parkinson's disease alongside L-DOPA. In a more preferred aspect of the invention, the L-DOPA is co-administered with an AADC inhibitor, such as benserazide or carbidopa.

**E. Process for determining the agglomerate distribution of crystalline opicapone (not claimed)**

As disclosed above, the inventors surprisingly discovered certain batches of pharmaceutical product consisting essentially of micronised crystalline opicapone were not bioequivalent when formulated into a final medicament or drug product in spite of fulfilling primary particle size restrictions according to standard ECD calculations (e.g., D10, D50, and

D90).

After extensive experimentation, the inventors discovered a technique for positioning a dry sample of the pharmaceutical product onto a solid surface that allowed the detection of previously-unknown agglomerated particles of crystalline opicapone.

Through optimisation of conditions, the inventors identified a reliable and reproducible process for determining the agglomerate distribution of a pharmaceutical product. The optimal conditions are detailed in Experiment 1 below.

As will be described below, the inventors identified two characteristic types of agglomerate - sheaf agglomerates and globular agglomerates. The presence of high amounts of sheaf agglomerates correlated with poor bioavailability and non-bioequivalence, whereas the presence of high amounts of globular agglomerates correlated with good bioavailability and bioequivalence.

Now that the inventors have identified the cause of the batch-to-batch variability and identified conditions in which different agglomerate forms can be distinguished, it will be possible to visualise and distinguish these agglomerates using alternative techniques. For example, the inventors have visualised these agglomerates using both light microscopy and scanning electron microscopy. It is envisaged that at least atomic force microscopy and more specialised forms of light scattering (e.g., calculating the shape factor $\rho$ and polydispersity using combined dynamic and static light scattering) may also be used.

Therefore, this disclosure describes a process for determining the agglomerate distribution of a pharmaceutical product consisting essentially of micronised crystalline opicapone comprising the steps of:

i) positioning a dry sample of the pharmaceutical product for agglomerate analysis without disaggregating the agglomerates;
ii) determining the percentage of sheaf agglomerates within the sample; and
iii) determining the percentage of globular agglomerates within the sample.

A convenient manner to position the dry sample is by the use of moderate pressure. This allows the sample to be positioned for agglomerate analysis without disaggregating the agglomerates. Therefore, the disclosure describes a process for determining the agglomerate distribution of a pharmaceutical product involves positioning the dry sample with the application of pressure.

The inventors found that dispersion of the pharmaceutical product in a way that separated the agglomerates but did not cause their disaggregation could be optimised by using particular application pressures and/or sample sizes. Therefore, in a more preferred embodiment the process for determining the agglomerate distribution of the pharmaceutical product involves positioning a dry sample of the pharmaceutical product for agglomerated analysis using an application pressure of between 0.1 bar and 2 bar, preferably between 0.5 bar and 1.5 bar, and more preferably between 1 bar. Pressures below this range did not result in correct positioning of larger amounts of the pharmaceutical product for agglomerate analysis, because the sample did not distribute sufficiently to visualise individual agglomerates. Pressures above this range could cause disaggregation of the agglomerates, especially the globular agglomerates, and especially when smaller amounts of the pharmaceutical product were analysed.

As described herein, the process for determining the agglomerate distribution of the pharmaceutical product may involve positioning a dry sample of the pharmaceutical product for agglomerate analysis using between 0.1 and 2 mg, preferably between 0.5 and 1.5 mg and more preferably about 1 mg of the dry pharmaceutical product. Amounts below this range were more sensitive to disaggregation of the agglomerate and amounts above this range were harder to distribute sufficiently to visualise individual agglomerates.

**F. Process for determining the primary particle size distribution of crystalline opicapone (not claimed)**

Once the inventors identified a suitable process for determining the agglomerate distribution of a pharmaceutical product, they proceeded to identify an orthogonal process for determining the primary particle size distribution of the pharmaceutical product, i.e., a process that fully disaggregated all agglomerates yet allowed the primary particles of micronised opicapone to remain intact.

After extensive experimentation, the inventors discovered a technique for dispersing the pharmaceutical product in mineral oil in a manner which disaggregates any agglomerates and then positioning the dispersion onto a solid surface that allows the measurement of the maximum distance and/or the total fibre length of single primary particles of crystalline opicapone.

Through optimisation of conditions, the inventors identified a reliable and reproducible process for determining the primary particle size distribution (i.e. maximum distance and/or total fibre length distribution) of a pharmaceutical product. The optimal conditions are detailed in Experiment 2 below.

Therefore, this disclosure describes a process for determining the primary particle size distribution of a pharmaceutical product consisting essentially of micronised crystalline opicapone comprising the steps of:

i) dispersing the pharmaceutical product in mineral oil in a manner which disaggregates any agglomerates;
ii) positioning the dispersion for particle size measurement;
iii) measuring the maximum distance between any two points of a single particle of crystalline opicapone;
iv) repeating step iii) for at least 100 particles; and
v) calculating the D10 (maximum distance), D50 (maximum distance) and D90 (maximum distance) values.

Given that the maximum distance of a particle directly and strongly correlates with the total fibre length, this disclosure describes a process for determining the primary particle size distribution of a pharmaceutical product consisting essentially of micronised crystalline opicapone comprising the steps of:

i) dispersing the pharmaceutical product in mineral oil in a manner which disaggregates any agglomerates;
ii) positioning the dispersion for particle size measurement;
iii) measuring the total fibre length of a single particle of crystalline opicapone;
iv) repeating step iii) for at least 100 particles; and
v) calculating the D10 (total fibre length), D50 (total fibre length) and D90 (total fibre length) values.

As described herein, the processes for determining the primary particle size distribution of the pharmaceutical product may involve dispersing a sample of the pharmaceutical product in mineral oil for particle size analysis using between 0.1 and 2 mg, preferably between 0.5 and 1.5 mg and more preferably about 1 mg of the dry pharmaceutical product. Amounts below this range were more sensitive to disaggregation of the agglomerate and amounts above this range were hardest to distribute sufficiently to visualise individual particles. It is clear to the skilled person, that larger or smaller amounts of pharmaceutical product in mineral oil could be utilised as long as their relative proportions and the concentration of the suspended pharmaceutical product remains within this range.

As described herein, the processes for determining the primary particle size distribution of the pharmaceutical product may involve detection using light microscopy and/or light scattering techniques light scattering (e.g., calculating the shape factor $\rho$ and polydispersity using combined dynamic and static light scattering). As described herein, the processes for determining the primary particle size distribution of the pharmaceutical product may involve detection using light microscopy.

G. Examples

Experiment 1 - "dry" process for identification of agglomerates and their characterisation

Measurements were carried out by the Morphologi G3 (MG3) method using Malvern equipment equipped with a sample dispersion unit plate and with the following instrumental parameters:

Sample amount: about 1 mg
SOP optic(s): 2.5x
Light source: Episcopic (top light)
Threshold: 0-78
Scan area: 64.5 x 49.0
Size bands: 81
Injection pressure: 1 bar
Fiber width < 14 μm
Circularity < 0.2

Additional information about the Morphologi technique and apparatus can be obtained from the manufacturer Malvern Panalytical or obtained from the following internet address https://www.malvernpanalytical.com/en/products/product-range/morphologi-range.

It was important to obtain a homogeneous dispersion of sample without fragmentation of agglomerates. This could be achieved by careful tuning of the sample amount (to facilitate dispersion on the glass slide) and injection pressure (to obtain a homogeneous dispersion without fragmentation of agglomerates).

Globular agglomerates were identified by the following classification:

Polygon: [solidity; aspect ratio]
([0.230;1];[0.820;0];[1;0];[1;1])
Elongation ≤ 0.550

Sheaf agglomerates were identified by the following classification:
Elongation > 0.550

Results from analysis of 5 comparative samples of micronized crystalline opicapone and 7 inventive samples of micronized crystalline opicapone are shown in **Table 3** below:

**Table 3**

| Parameter / Examp. | Total no. of agglomerates | No. of sheaf agglomerates | No. of globular agglomerates | % sheaf agglomerates | % globular agglomerates |
|---|---|---|---|---|---|
| Comparative 1 | 109 | 30 | 79 | 28 | 72 |
| Comparative 2 | 119 | 42 | 77 | 35 | 65 |
| Comparative 3 | 116 | 39 | 77 | 34 | 66 |
| Comparative 4 | 59 | 26 | 32 | 44 | 56 |
| Comparative 5 | 234 | 43 | 191 | 18 | 82 |
| Invention 1 | 469 | 29 | 440 | 6 | 94 |
| Invention 2 | 316 | 18 | 298 | 6 | 94 |
| Invention 3 | 291 | 20 | 271 | 7 | 93 |
| Invention 4 | 355 | 47 | 309 | 13 | 87 |
| Invention 5 | 307 | 16 | 291 | 5 | 95 |
| Invention 6 | 169 | 6 | 163 | 4 | 96 |
| Invention 7 | 210 | 9 | 201 | 4 | 96 |

Experiment 2 - "wet" process for determining the primary particle size distribution of a pharmaceutical product

Approximately 2 mg of crystalline opicapone was accurately weighed and then transferred into a beaker containing mineral oil. An appropriate quantity of the prepared suspension was then collected, spread on a microscope slide and covered with a coverslip.

Measurements of maximum distance and/or total fibre length were carried out using the MG3 method with the following instrumental parameters:

SOP optic(s): 10x
Light source: Diascopic (bottom light)
Threshold: 0-174
Scan area: 15x25 mm
Size bands: 81
Filters: Convexity ≤ 0.7
Intensity SD ≥ 25

Results from analysis of 3 of the above comparative samples of micronized crystalline opicapone and 5 of the above inventive samples of micronized crystalline opicapone are shown in **Table 4** below:

**Table 4**

| Example / Param. | Comparative 1 | Comparative 3 | Comparative 4 | Invention 3 | Invention 1 | Invention 2 | Invention 5 | Invention 4 |
|---|---|---|---|---|---|---|---|---|
| FTL D10 | 17 | 22 | 26 | 14 | 13 | 15 | 15 | 11 |
| FTL D50 | 66 | 94 | 131 | 49 | 42 | 39 | 35 | 36 |
| FTL D90 | 230 | 290 | 421 | 190 | 165 | 111 | 80 | 142 |
| FTL D95 | 285 | 360 | 546 | 289 | 240 | 216 | 107 | 227 |
| MD D10 | 16 | 20 | 24 | 14 | 13 | 14 | 14 | 12 |
| MD D50 | 60 | 85 | 117 | 44 | 37 | 34 | 32 | 32 |
| MD D90 | 198 | 239 | 340 | 154 | 131 | 87 | 66 | 120 |
| MD D95 | 243 | 301 | 433 | 232 | 191 | 180 | 85 | 184 |

FTL = Fibre Total Length (units are μm) MD = Maximum Distance (units are μm)

Experiment 3 - milling and/or re-milling of pharmaceutical product

Milling of crystalline opicapone was carried out using an MC JETMILL®200 micronizer. Several trials were conducted to identify optimum milling conditions. A feed rate of 150 g/30 sec and a milling pressure of 6.0 bar were selected as optimum milling conditions. The results of re-milling non-compliant micronized crystalline opicapone (Comparative Examples 2 and 3 above) under these conditions are shown in **Tables 5 and 6** below:

**Table 5**

| Parameter / Examp. | Total no. of agglomerates | No. of sheaf agglomerates | No. of globular agglomerates | % sheaf agglomerates | % globular agglomerates |
|---|---|---|---|---|---|
| Comparative 2 | 119 | 42 | 77 | 35 | 65 |
| Invention ( = Re-milled Comparative 2) | 187 | 10 | 177 | 5 | 95 |
| Comparative 3 | 116 | 39 | 77 | 34 | 66 |
| Invention ( = Re-milled Comparative 3) | 187 | 10 | 177 | 5 | 95 |

**Table 6**

| Parameter / Examp. | FTL D10 | FTL D50 | FTL D90 | FTL D95 | MD D10 | MD D50 | MD D90 | MD D95 |
|---|---|---|---|---|---|---|---|---|
| Comparative 2 | 20 | 79 | 263 | 345 | 18 | 70 | 218 | 273 |
| Invention ( = Re-milled Comparative 2) | 10 | 32 | 122 | 167 | 10 | 29 | 105 | 143 |
| Comparative 3 | 22 | 94 | 290 | 360 | 20 | 85 | 239 | 301 |
| Invention ( = Re-milled Comparative 3) | 11 | 46 | 180 | 238 | 11 | 42 | 148 | 195 |

Experiment 4 - bioavailability experiments on different batches of pharmaceutical product

*4. 1 Bioavailability in rats*

**General procedure**

During the studies, blood was collected at different time points, from tail vein, spun at 1500 x g in a refrigerated centrifuge (4°C) for 15 min, and the plasma obtained was stored at -80°C until further analysis. The plasma samples collected from thirty animals (270 samples), were analysed for opicapone exposure. The bioanalysis involved the use of LC-MS/MS after plasma precipitation.

**Tested materials**

Studies were conducted using pharmaceutical product which was (i) not in accordance with the invention (Comparative 3), (ii) in accordance with the invention (Invention 3 + Invention 1), and (iii) the same as that used in study (i) but re-milled to convert it into product in accordance with the invention (Re-milled Comparative 3).

**Results**

(i) Following a single oral administration of micronized crystalline opicapone (50 mg suspended in 100 ml HPMC 0.2%) to male Wistar rats, at a target dose level of 3 mg/kg,
the mean concentration of opicapone in plasma was detectable shortly after administration ($T_{max}$ range between 1 to 3 h post-dose) with a $C_{max}$ of 508.4 (62.5) ng/mL and an $AUC_{(0\text{-last})}$ of 1209.4 (55.4) ng*h/mL (n = 10).
(ii) Following a single oral administration of micronized crystalline opicapone (50 mg suspended in 100 ml HPMC 0.2%) to male Wistar rats, at a target dose level of 3 mg/kg,
the mean concentration of opicapone in plasma was detectable shortly after administration ($T_{max}$ range between 1 to 3 h post-dose) with a $C_{max}$ of 827.1 (55.9) ng/mL and an $AUC_{(0\text{-last})}$ of 2266.5 (36.0) ng*h/mL (n = 10).
(iii) Following a single oral administration of micronized crystalline opicapone (50 mg suspended in 100 ml HPMC 0.2%) to male Wistar rats, at a target dose level of 3 mg/kg,
the mean concentration of opicapone in plasma was detectable shortly after administration ($T_{max}$ range between 1 to 3 h post-dose) with a $C_{max}$ of 1009.6 (46.7) ng/mL and an $AUC_{(0\text{-last})}$ of 2193.7 (37.3) ng*h/mL (n = 10).

**Conclusions**

Micronised crystalline opicapone which was already in accordance with the claimed invention (ii) or which was re-milled to bring it into accordance with the claimed invention (iii) exhibited similar bioavailability which was much greater

than that exhibited by micronized crystalline opinapone which was not in accordance with the claimed invention (see **Figure 9).**

*4.2 Bioavailability in humans*

**General procedure and tested materials**

An open-label, 3-period, 3-sequence, partial-replicate crossover clinical study, wherein the reference opicapone source (drug product containing pharmaceutical product in accordance with the present invention) was administered twice, and the test opicapone source (drug product containing pharmaceutical product originally not in accordance with the present invention but re-milled to convert it into product in accordance with the invention) was administered once. This allowed the assessment of the within subject variability of the reference source. The crossover design chosen for this study enabled subjects to act as their own control. Treatment sequence randomisation prevented any selection bias that might otherwise have resulted from treatment order. Furthermore, as exposure to opicapone is significantly reduced when administered in the fed state, the bioequivalence was evaluated under fasting conditions after single-dose administration. These were also considered to be the most sensitive conditions to detect a potential difference between the two opicapone sources.

**Results**

In this clinical study, drug product manufactured with re-milled crystalline opicapone (test) and compliant crystalline opicapone (reference) was found to be bioequivalent at 50 mg strength, with the 90% CIs of the GMRs for $AUC_{0-t}$ (105.32-117.13) and $C_{max}$ (108.42-124.42) within the bioequivalence acceptance range of 80.00% to 125.00% (see **Table 7).**

**Table 7**

| Parameter | Treatment | n | Geometric Mean | Geometric Mean Ratio (%) | | Within-subject CV% |
|---|---|---|---|---|---|---|
| | | | | Estimate | 90% CI | |
| $AUC_{0-t}$ (h*ng/mL) | Test | 44 | 2850 | 111.07 | 105.32-117.13 | 18.99 |
| | Reference | 88 | 2570 | | | |
| $C_{max}$ (ng/mL) | Test | 44 | 1100 | 116.06 | 108.42-124.24 | 24.04 |
| | Reference | 88 | 945 | | | |

**Conclusion**

Drug product made from micronised crystalline opicapone which was already in accordance with the claimed invention (reference) was bioequivalent to that made from micronised crystalline opicapone which was re-milled to bring it into accordance with the claimed invention (test).

<u>Formulation Examples</u>

The pharmaceutical product of the present invention may be combined with one or more pharmaceutically acceptable excipients to form a pharmaceutical composition suitable for oral administration. Preferably, the method involves the formation of granules of the pharmaceutical product and the one or more excipients. More preferably, the method involves formation of a unit dose of the granules. Even more preferably, the unit dose is a capsule or a tablet.

In one exemplary embodiment, the pharmaceutical composition comprises 0.2 to 50 wt% pharmaceutical product and 50 to 99.8 wt% of pharmaceutically acceptable excipient(s), preferably comprising 1 to 15 wt% binder and 33 to 85 wt% filler, and optionally 0.5 to 15 wt% lubricant and/or 1 to 15 wt% disintegrant, such as the following compositions and/or formulations:

| | |
|---|---|
| Pharmaceutical product (of the present invention) | 0.2 - 50 wt% |
| Filler | 35.0 - 85.0 wt% |
| Binder | 1.0 - 15.0 wt% |
| Lubricant | 1.0 - 15.0 wt% |

(continued)

| | |
|---|---|
| Disintegrant | 1.0 - 15.0 wt % |

| | |
|---|---|
| Pharmaceutical product (of the present invention) | 30.0 - 50.0 wt% |
| Filler | 35.0 - 60.0 wt% |
| Binder | 3.0 - 10.0 wt% |
| Lubricant | 1.0 - 10.0 wt% |
| Disintegrant | 3.0 - 10.0 wt% |

| | |
|---|---|
| Pharmaceutical product (of the present invention) | 0.2 - 35 wt% |
| Filler | 50.0 - 85.0 wt% |
| Binder | 3.0 - 10.0 wt% |
| Lubricant | 1.0 - 10.0 wt% |
| Disintegrant | 3.0 - 10.0 wt% |

| | |
|---|---|
| Pharmaceutical product (of the present invention) | 5 - 25 wt% |
| Filler | 60.0 - 80.0 wt% |
| Binder | 5.0 - 10.0 wt% |
| Lubricant | 0.5 - 4.0 wt% |
| Disintegrant | 4.0 - 8.0 wt% |

Such pharmaceutical compositions may be in the form of a dosage form such as a capsule or a compressed form such as a tablet.

Fillers/diluents of the present disclosure include calcium phosphate, dibasic anhydrous (for example, A-TAB ™, Di-Cafos A-N ™, Emcompress ™ Anhydrous, and Fujicalin ™); calcium phosphate, dibasic dihydrate (for example, Cafos ™, Calipharm ™, Calstar ™, Di-Cafos ™, Emcompress ™); and calcium phosphate tribasic (for example, Tri-Cafos ™, TRI-CAL ™ WG, TRI-TAB ™). In a further embodiment, the filler may be chosen from starches, lactose, and cellulose. In at least one embodiment, at least two fillers may be present, for example a combination of starch, lactose, and/or cellulose. Preferred filler is lactose.

Binders of the present disclosure include acacia, alginic acid, carbomer, carboxymethylcellulose sodium, ceratonia, cottonseed oil, dextrin, dextrose, gelatin, guar gum, hydrogenated vegetable oil type I, hydroxyethyl cellulose, hydroxyethylmethyl cellulose, hydroxypropyl cellulose, low substituted hydroxypropyl cellulose, hypromellose, magnesium aluminium silicate, maltodextrin, maltose, methylcellulose, ethylcellulose, microcrystalline cellulose, polydextrose, polyethylene oxide, polymethacrylates, sodium alginate, starch, pregelatinised starch, stearic acid, sucrose and zein. Preferred binder is pregelatinised starch.

Lubricants/flow agents of the present disclosure include calcium stearate, glycerine monostearate, glyceryl behenate, glyceryl palmitostearate, hydrogenated castor oil, hydrogenated vegetable oil type I, magnesium lauryl sulphate, magnesium stearate, medium-chain triglycerides, poloxamer, polyethylene glycol, sodium benzoate, sodium chloride, sodium lauryl sulphate, sodium stearyl fumarate, stearic acid, talc, sucrose stearate, and zinc stearate, and mixtures thereof. Preferred lubricant is magnesium stearate.

Suitable disintegrants of the present disclosure include agar, calcium carbonate, alginic acid, calcium phosphate (tribasic), carboxymethylcellulose calcium, carboxymethylcellulose sodium, colloidal silicon dioxide, croscarmellose sodium, crospovidone, docusate sodium, guar gum, low substituted hydroxypropyl cellulose, magnesium aluminium silicate, methylcellulose, microcrystalline cellulose, sodium alginate, sodium starch glycolate, polacrilin potassium, silicified microcrystalline cellulose, starch and pre-gelatinized starch, and mixtures thereof. The disintegrant may be a combination of disintegrants and/or at least two disintegrants are present, for example a combination of sodium carboxymethyl starch and sodium starch glycolate, such as the sodium starch glycolate sold under the trade name Explotab™. The preferred disintegrant is sodium starch glycolate, in particular Explotab™.

Further examples of pharmaceutical compositions suitable for the preparation of 25 mg and 50 mg strength capsules and tablets of opicapone (BIA 9-1067) are provided in Tables 8 and 9 below:

## Table 8

| | Dosage form[a] 1 | | Dosage form[a] 2 | | Dosage form[a] 3 | | Dosage form[a] 4 | | Dosage form[a] 5 | | Dosage form[a] 6 | | Dosage form[a] 7 | | Dosage form[a] 8 | | |
|---|---|---|---|---|---|---|---|---|---|---|---|---|---|---|---|---|---|
| Starting material | mg | % | mg | % | mg | % | mg | % | mg | % | mg | % | mg | % | mg | % | Function |
| BIA 9-1067 | 25 | 10,6 | 25 | 10,6 | 25 | 10,4 | 25 | 10,4 | 25 | 10,2 | 25 | 10,2 | 25 | 10 | 25 | 10 | Active substance |
| Lactose | 176 | 74,9 | 173 | 73,6 | 183 | 76,3 | 175 | 72,9 | 181 | 73,9 | 179 | 73,1 | 181 | 72,4 | 186 | 74,4 | Diluent |
| Pregelatinized starch | 15 | 6,4 | 18 | 7,7 | 16 | 6,7 | 22 | 9,2 | 17 | 6,9 | 14 | 5,7 | 19 | 7,6 | 17 | 6,8 | Binder |
| Purified water[b] | q.s. | | q.s. | | q.s. | | q.s. | | q.s. | | q.s. | | q.s. | | q.s. | | Granulation liquid |
| Sodium starch glycolate | 15 | 6,4 | 13 | 5,5 | 10 | 4,2 | 16 | 6,7 | 17 | 6,9 | 19 | 7,8 | 18 | 7,2 | 19 | 7,6 | Disintegrant |
| Magnesium stearate | 4 | 1,7 | 6 | 2,6 | 6 | 2,5 | 2 | 0,8 | 5 | 2 | 8 | 3,3 | 7 | 2,8 | 3 | 1,2 | Flow agent |

[a] Composition per dosage form (e.g. capsule, tablet, etc). Tablets may be film-coated (e.g. with, celulose derivates, starch derivates, acrylic acid derivates, PVA (polyvinyl alcohol) or Polyvinyl Acetate Phthalate based film coatings)

[b] Not present in final product (only used if wet granulation is the chosen method)

q.s. - sufficient quantity

## Table 9

| | Dosage form[a] 1 | | Dosage form[a] 2 | | Dosage form[a] 3 | | Dosage form[a] 4 | | Dosage form[a] 5 | | Dosage form[a] 6 | | Dosage form[a] 7 | | Dosage form[a] 8 | | |
|---|---|---|---|---|---|---|---|---|---|---|---|---|---|---|---|---|---|
| Starting material | mg | % | mg | % | mg | % | mg | % | mg | % | mg | % | mg | % | mg | % | Function |
| BIA 9-1067 | 50 | 21,3 | 50 | 21,3 | 50 | 20,8 | 50 | 20,8 | 50 | 20,4 | 50 | 20,4 | 50 | 20 | 50 | 20 | Active substance |
| Lactose | 151 | 64,3 | 148 | 63 | 158 | 65.8 | 150 | 62.5 | 156 | 63.7 | 154 | 62,9 | 156 | 62,4 | 161 | 64.4 | Diluent |
| Pregelatinized starch | 15 | 6,4 | 18 | 7,7 | 16 | 6,7 | 22 | 9,2 | 17 | 6,9 | 14 | 5,7 | 19 | 7,6 | 17 | 6,8 | Binder |
| Purified water[b] | q.s. | | q.s. | | q.s. | | q.s. | | q.s. | | q.s. | | q.s. | | q.s. | | Granulation liquid |
| Sodium starch glycolate | 15 | 6,4 | 13 | 5,5 | 10 | 4,2 | 16 | 6,7 | 17 | 6,9 | 19 | 7,3 | 18 | 7,2 | 19 | 7,6 | Disintegrant |
| Magnesium stearate | 4 | 1,7 | 6 | 2,6 | 6 | 2,5 | 2 | 0,8 | 5 | 2 | 8 | 3,3 | 7 | 2,8 | 3 | 1,2 | Flow agent |

[a] Composition per dosage form (e.g. capsule, tablet, etc). Tablets may be film-coated (e.g. with, celulose derivates, starch derivates, acrylic acid derivates, PVA (polyvinyl alcohol) or Polyvinyl Acetate Phthalate based film coatings)

[b] Not present in final product (only used if wet granulation is the chosen method)

q.s. - sufficient quantity

## Claims

1. A pharmaceutical product consisting essentially of crystalline opicapone having the following primary particle size distribution as defined by maximum distance:

   D10 greater than or equal to 8 μm;
   D50 of 20 to 55 μm; and
   D90 less than or equal to 200 μm;

   and the following agglomerate distribution:

   % number of sheaf agglomerates less than or equal to 25%;
   wherein crystalline opicapone comprises at least 95 wt% of the total dry weight of the pharmaceutical product;
   wherein the term "sheaf agglomerate" is defined as measured in Experiment 1 of the description;
   wherein the "maximum distance" and "primary particle size distribution" are measured as described in Experiment 2 of the description; and
   wherein the term "agglomerate" refers to an assemblage of at least 10 primary particles of crystalline opicapone.

2. The pharmaceutical product according to claim 1, wherein the crystalline opicapone has a % number of globular agglomerates greater than or equal to 80%, preferably greater than or equal to 90%; wherein the term "globular agglomerate" is defined as measured in Experiment 1 of the description.

3. The pharmaceutical product according to any one of claims 1 to 2, wherein the total area occupied by sheaf agglomerates in a 1 mg sample, as determined by particle size measurement, is less than 4.0 x $10^6$ $\mu m^2$/mg, preferably less than 3.0 x $10^6$ $\mu m^2$/mg, more preferably less than 2.0 x $10^6$ $\mu m^2$/mg, even more preferably less than 1.0 x $10^6$ $\mu m^2$/mg.

4. The pharmaceutical product according to any one of claims 1 to 3, wherein the crystalline opicapone has the following primary particle size distribution as defined by maximum distance:

   i) D10 greater than or equal to 9 μm;
   ii) D50 of 25 to 50 μm; and
   iii) D90 less than or equal to 180 μm.

5. The pharmaceutical product according to any one of claims 1 to 4, wherein crystalline opicapone comprises at least 97 wt%, preferably at least 99 wt%, of the total dry weight of the pharmaceutical product.

6. A pharmaceutical product comprising the pharmaceutical product according to any one of claims 1 to 5 and one or more pharmaceutically acceptable excipients, preferably wherein the pharmaceutical product is in the form of granules.

7. A pharmaceutical product comprising the pharmaceutical product in the form of granules of claim 6 and one or more pharmaceutically acceptable excipients.

8. A capsule or tablet for oral administration comprising a pharmaceutical product according to any one of claims 6 or 7.

9. A method of manufacturing a pharmaceutical product comprising the following steps:

   a) micronising a product consisting essentially of crystalline opicapone;
   b) determining the primary particle size distribution and the % number of sheaf agglomerates for the crystalline opicapone in the micronized product;
   c) retaining micronized product consisting essentially of crystalline opicapone having the following primary particle size distribution as defined by maximum distance:

      D10 greater than or equal to 8 μm;
      D50 of 20 to 55 μm; and
      D90 less than or equal to 200 μm;

and the following agglomerate distribution:
% number of sheaf agglomerates less than or equal to 25%; and
d) if necessary, repeating steps a) to c) on micronized product consisting essentially of crystalline opicapone which does not have the primary particle size and agglomerate distributions defined in step c) above;
wherein crystalline opicapone comprises at least 95 wt% of the total dry weight of the pharmaceutical product;
wherein the term "sheaf agglomerate" is defined as measured in Experiment 1 of the description;
wherein the "maximum distance" and "primary particle size distribution" are measured as described in Experiment 2 of the description; and
wherein the term "agglomerate" refers to an assemblage of at least 10 primary particles of crystalline opicapone.

**10.** The method according to claim 9, wherein the pharmaceutical product of step c) has a % number of globular agglomerates greater than or equal to 80%, preferably greater than or equal to 90%; wherein the term "globular agglomerate" is defined as measured in Experiment 1 of the description.

**11.** The method of manufacturing a pharmaceutical product according to any one of claims 9 or 10, wherein the crystalline opicapone has the following primary particle size distribution as defined by maximum distance:

ii) D10 greater than or equal to 9 μm;
ii) D50 of 25 to 50 μm; and
iii) D90 less than or equal to 180 μm.

**12.** The method according to any one of claims 9 to 11, wherein the pharmaceutical product of step c) is combined with one or more pharmaceutically acceptable excipients.

**13.** A medicament comprising a pharmaceutical product as defined in any one of claims 1 to 7, for use in increasing opicapone bioavailability in a patient suffering from Parkinson's disease, as compared to the opicapone bioavailability which would be obtained from an equivalent medicament comprising a pharmaceutical product as defined in any one of claims 1 to 7 except for having a % number of sheaf agglomerates greater than 30%.

**14.** The medicament for use according to claim 13, wherein opicapone bioavailability is increased by at least 10% and more preferably, at least 20%.

**15.** The medicament for use according to claim 13 or 14, wherein the patient suffering from Parkinson's disease is co-administered levodopa.

**Patentansprüche**

**1.** Pharmazeutisches Produkt, bestehend im Wesentlichen aus kristallinem Opicapon mit der folgenden Primärpartikelgrößenverteilung, wie definiert durch den maximalen Abstand:

D10 größer als oder gleich 8 μm;
D50 von 20 bis 55 μm; und
D90 kleiner als oder gleich 200 μm;
und der folgenden Agglomeratverteilung:

Prozentzahl an garbenförmigen Agglomeraten kleiner als oder gleich 25 %;
wobei kristallines Opicapon mindestens 95 Gew.-% des Gesamttrockengewichts des pharmazeutischen Produkts umfasst;
wobei der Begriff "garbenförmiges Agglomerat" wie in Experiment 1 der Beschreibung gemessen definiert ist;
wobei der "maximale Abstand" und die "Primärpartikelgrößenverteilung" wie in Experiment 2 der Beschreibung beschrieben gemessen werden; und
wobei der Begriff "Agglomerat" eine Anordnung von mindestens 10 Primärpartikeln von kristallinem Opicapon bezeichnet.

**2.** Pharmazeutisches Produkt gemäß Anspruch 1, wobei das kristalline Opicapon eine Prozentzahl von globulären Agglomeraten aufweist, die größer als oder gleich 80 %, vorzugsweise größer als oder gleich 90 % ist; wobei der

Begriff "globuläres Agglomerat" wie in Experiment 1 der Beschreibung gemessen definiert ist.

**3.** Pharmazeutisches Produkt gemäß einem beliebigen der Ansprüche 1 bis 2, wobei die durch Partikelgrößenmessung bestimmte Gesamtfläche, die von garbenförmigen Agglomeraten in einer 1-mg-Probe eingenommen wird, weniger als 4,0 x $10^6$ $\mu m^2$/mg, vorzugsweise weniger als 3,0 x $10^6$ $\mu m^2$/mg, stärker bevorzugt weniger als 2,0 x $10^6$ $\mu m^2$/mg, noch stärker bevorzugt weniger als 1,0 x $10^6$ $\mu m^2$/mg beträgt.

**4.** Pharmazeutisches Produkt gemäß einem beliebigen der Ansprüche 1 bis 3, wobei das kristalline Opicapon die folgende Primärpartikelgrößenverteilung aufweist, wie definiert durch den maximalen Abstand:

i) D10 größer als oder gleich 9 µm;
ii) D50 von 25 bis 50 µm; und
iii) D90 kleiner als oder gleich 180 µm.

**5.** Pharmazeutisches Produkt gemäß einem beliebigen der Ansprüche 1 bis 4, wobei kristallines Opicapon mindestens 97 Gew.-%, vorzugsweise mindestens 99 Gew.-%, des Gesamttrockengewichts des pharmazeutischen Produkts umfasst.

**6.** Pharmazeutisches Produkt, umfassend das pharmazeutische Produkt gemäß einem beliebigen der Ansprüche 1 bis 5 und einen oder mehrere pharmazeutisch unbedenkliche Hilfsstoffe, wobei vorzugsweise das pharmazeutische Produkt in Form von Granulat vorliegt.

**7.** Pharmazeutisches Produkt, umfassend das pharmazeutische Produkt in Form von Granulat nach Anspruch 6 und einen oder mehrere pharmazeutisch unbedenkliche Hilfsstoffe.

**8.** Kapsel oder Tablette zur oralen Verabreichung, umfassend ein pharmazeutisches Produkt gemäß einem beliebigen der Ansprüche 6 oder 7.

**9.** Verfahren zum Herstellen eines pharmazeutischen Produkts, umfassend die folgenden Schritte:

a) Mikronisieren eines Produkts, das im Wesentlichen aus kristallinem Opicapon besteht;
b) Bestimmen der Primärpartikelgrößenverteilung und der Prozentzahl der garbenförmigen Agglomerate für das kristalline Opicapon in dem mikronisierten Produkt;
c) Zurückhalten eines mikronisierten Produkts, im Wesentlichen bestehend aus kristallinem Opicapon mit der folgenden Primärpartikelgrößenverteilung, wie definiert durch den maximalen Abstand:

D10 größer als oder gleich 8 µm;
D50 von 20 bis 55 µm; und
D90 kleiner als oder gleich 200 µm;
und der folgenden Agglomeratverteilung:
Prozentzahl an garbenförmigen Agglomeraten kleiner als oder gleich 25 %; und

d) gegebenenfalls Wiederholen der Schritte a) bis c) an mikronisiertem Produkt, das im Wesentlichen aus kristallinem Opicapon besteht, das nicht die in Schritt c) oben definierte Primärpartikelgrößen- und Agglomeratverteilungen aufweist;
wobei kristallines Opicapon mindestens 95 Gew.-% des Gesamttrockengewichts des pharmazeutischen Produkts umfasst;
wobei der Begriff "garbenförmiges Agglomerat" wie in Experiment 1 der Beschreibung gemessen definiert ist;
wobei der "maximale Abstand" und die "Primärpartikelgrößenverteilung" wie in Experiment 2 der Beschreibung beschrieben gemessen werden; und
wobei der Begriff "Agglomerat" eine Anordnung von mindestens 10 Primärpartikeln von kristallinem Opicapon bezeichnet.

**10.** Verfahren gemäß Anspruch 9, wobei das pharmazeutische Produkt von Schritt c) eine Prozentzahl von globulären Agglomeraten aufweist, die größer als oder gleich 80 %, vorzugsweise größer als oder gleich 90 % ist; wobei der Begriff "globuläres Agglomerat" wie in Experiment 1 der Beschreibung gemessen definiert ist.

**11.** Verfahren zum Herstellen eines pharmazeutischen Produkts gemäß einem beliebigen der Ansprüche 9 bis 10, wobei

das kristalline Opicapon die folgende Primärpartikelgrößenverteilung aufweist, wie definiert durch den maximalen Abstand:

ii) D10 größer als oder gleich 9 μm;
ii) D50 von 25 bis 50 μm; und
iii) D90 kleiner als oder gleich 180 μm.

**12.** Verfahren gemäß einem beliebigen der Ansprüche 9 bis 11, wobei das pharmazeutische Produkt von Schritt c) mit einem oder mehreren pharmazeutisch verträglichen Hilfsstoffen kombiniert wird.

**13.** Arzneimittel, umfassend ein pharmazeutisches Produkt wie in einem der Ansprüche 1 bis 7 definiert, zur Verwendung bei der Erhöhung der Opicapon-Bioverfügbarkeit bei einem Patienten, der an Parkinson-Krankheit leidet, im Vergleich zu der Opicapon-Bioverfügbarkeit, die mit einem äquivalenten Arzneimittel erhalten würde, umfassend ein pharmazeutisches Produkt wie in einem der Ansprüche 1 bis 7 definiert, außer dass es eine Prozentzahl von garbenförmigen Agglomeraten von mehr als 30 % aufweist.

**14.** Arzneimittel zur Verwendung gemäß Anspruch 13, wobei die Opicapon-Bioverfügbarkeit um mindestens 10 % und stärker bevorzugt um mindestens 20 % erhöht ist.

**15.** Arzneimittel zur Verwendung gemäß Anspruch 13 oder 14, wobei dem an Parkinson-Krankheit leidenden Patienten gleichzeitig Levodopa verabreicht wird.

## Revendications

**1.** Produit pharmaceutique constitué essentiellement d'opicapone cristalline présentant la distribution granulométrique de particules primaires suivante telle que définie par une distance maximale :

D10 supérieur ou égal à 8 μm ;
D50 de 20 à 55 μm ; et
D90 inférieur ou égal à 200 μm ;
et la distribution d'agglomérats suivante :

% en nombre d'agglomérats en gerbe inférieur ou égal à 25 % ;
dans lequel l'opicapone cristalline comprend au moins 95 % en poids du poids sec total du produit pharmaceutique ;
dans lequel le terme « aggloméral en gerbe » est défini tel que mesuré dans l'Expérience 1 de la description ;
dans lequel la « distance maximale » et la « distribution granulométrique de particules primaires » sont mesurées comme décrit dans l'Expérience 2 de la description ; et
dans lequel le terme « agglomérat » fait référence à un assemblage d'au moins 10 particules primaires d'opicapone cristalline.

**2.** Produit pharmaceutique selon la revendication 1, dans lequel l'opicapone cristalline présente un % en nombre d'agglomérats globulaires supérieur ou égal à 80 %, de préférence supérieur ou égal à 90 % ; dans lequel le terme « agglomérat globulaire » est défini tel que mesuré dans l'Expérience 1 de la description.

**3.** Produit pharmaceutique selon l'une quelconque des revendications 1 à 2, dans lequel la surface totale occupée par des agglomérats en gerbe dans un échantillon de 1 mg, telle que déterminée par mesure de la taille des particules, est inférieure à 4,0 x $10^6$ $\mu m^2$/mg, de préférence inférieure à 3,0 x $10^6$ $\mu m^2$/mg, idéalement inférieure à 2,0 x $10^6$ $\mu m^2$/mg, encore idéalement inférieure à 1,0 x $10^6$ $\mu m^2$/mg.

**4.** Produit pharmaceutique selon l'une quelconque des revendications 1 à 3, dans lequel l'opicapone cristalline présente la distribution granulométrique de particules primaires suivante telle que définie par une distance maximale :

i) D10 supérieur ou égal à 9 μm ;
ii) D50 de 25 à 50 μm ; et
iii) D90 inférieur ou égal à 180 μm.

5. Produit pharmaceutique selon l'une quelconque des revendications 1 à 4, dans lequel l'opicapone cristalline comprend au moins 97 % en poids, de préférence au moins 99 % en poids, du poids sec total du produit pharmaceutique.

6. Produit pharmaceutique comprenant le produit pharmaceutique selon l'une quelconque des revendications 1 à 5 et un ou plusieurs excipients acceptables pharmaceutiquement, de préférence dans lequel le produit pharmaceutique est sous forme de granulés.

7. Produit pharmaceutique comprenant le produit pharmaceutique sous forme de granulés de la revendication 6 et un ou plusieurs excipients acceptables pharmaceutiquement.

8. Capsule ou comprimé pour administration orale comprenant un produit pharmaceutique selon l'une quelconque des revendications 6 ou 7.

9. Procédé de fabrication d'un produit pharmaceutique comprenant les étapes suivantes :

   a) micronisation d'un produit constitué essentiellement d'opicapone cristalline ;
   b) détermination de la distribution granulométrique de particules primaires et du % en nombre d'agglomérats en gerbe de l'opicapone cristalline dans le produit micronisé ;
   c) rétention d'un produit micronisé constitué essentiellement d'opicapone cristalline présentant la distribution granulométrique de particules primaires suivante telle que définie par une distance maximale :

      D10 supérieur ou égal à 8 μm ;
      D50 de 20 à 55 μm ; et
      D90 inférieur ou égal à 200 μm ;
      et la distribution d'agglomérats suivante :
      % en nombre d'agglomérats en gerbe inférieur ou égal à 25 % ; et

   d) si nécessaire, répétition des étapes a) à c) sur un produit micronisé constitué essentiellement d'opicapone cristalline qui ne présente pas la distribution granulométrie de particules primaires et la distribution d'agglomérats définies à l'étape c) ci-dessus ;
   dans lequel l'opicapone cristalline comprend au moins 95 % en poids du poids sec total du produit pharmaceutique ;
   dans lequel le terme « agglomérat en gerbe » est défini tel que mesuré dans l'Expérience 1 de la description ;
   dans lequel la « distance maximale » et la « distribution granulométrique de particules primaires » sont mesurées comme décrit dans l'Expérience 2 de la description ; et
   dans lequel le terme « agglomérat » fait référence à un assemblage d'au moins 10 particules primaires d'opicapone cristalline.

10. Procédé selon la revendication 9, dans lequel le produit pharmaceutique de l'étape c) présente un % en nombre d'agglomérats globulaires supérieur ou égal à 80 %, de préférence supérieur ou égal à 90 % ; dans lequel le terme « agglomérat globulaire » est défini tel que mesuré dans l'Expérience 1 de la description.

11. Procédé de fabrication d'un produit pharmaceutique selon l'une quelconque des revendications 9 ou 10, dans lequel l'opicapone cristalline présente la distribution granulométrique de particules primaires suivante telle que définie par une distance maximale :

   i) D10 supérieur ou égal à 9 μm ;
   ii) D50 de 25 à 50 μm ; et
   iii) D90 inférieur ou égal à 180 μm.

12. Procédé selon l'une quelconque des revendications 9 à 11, dans lequel le produit pharmaceutique de l'étape c) est combiné avec un ou plusieurs excipients acceptables pharmaceutiquement.

13. Médicament comprenant un produit pharmaceutique tel que défini dans l'une quelconque des revendications 1 à 7, destiné à être utilisé pour augmenter la biodisponibilité de l'opicapone chez un patient atteint de la maladie de Parkinson, par rapport à la biodisponibilité de l'opicapone qui serait obtenue à partir d'un médicament équivalent comprenant un produit pharmaceutique tel que défini dans l'une quelconque des revendications 1 à 7, à l'exception du

fait que celui-ci présente un % en nombre d'agglomérats en gerbe supérieur à 30 %.

**14.** Médicament destiné à être utilisé selon la revendication 13, dans lequel la biodisponibilité de l'opicapone est augmentée d'au moins 10 % et, idéalement, d'au moins 20 %.

**15.** Médicament destiné à être utilisé selon la revendication 13 ou 14, dans lequel la lévodopa est co-administrée au patient atteint de la maladie de Parkinson.

Figure 1

200 µm
100 µm

Figure 2

1000 µm
100 µm

Figure 3

Aspect Ratio
0
0.1
0.2
0.3
0.4
0.5
0.6
0.7
0.8
0.9
1
Solidity

Figure 4

A
A
(a)

Figure 5

(b)

Figure 6

(c)

Figure 7

Correlation between total fibre length and maximum distance
Maximum distance (µm)
500
400
300
200
100
0
0
100
200
300
400
500
600
Total fibre length (µm)
y = 0.8033x + 3.6859
R² = 0.9983

Figure 8

Correlation between Sheaf agglomerates and globular agglomerates
Sheaf agglomerates (%)
50
40
30
20
10
0
50
60
70
80
90
100
globular agglomerates (%)

Figure 9

1000
800
600
400
200
0
ng/ml
Comparative 3
Invention 3 + Invention 1
Re-milled Comparative 3
0
8
16
24
Time (h)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007013830 A
- WO 2009116882 A
- WO 2010114404 A
- WO 2010114405 A
- WO 2013089573 A


### Non-patent literature cited in the description


- **L. E. KISS et al.** *J. Med. Chem.*, 2010, vol. 53, 3396-3411
- **R. LIMA et al.** Relative Bioavailability of Opicapone from Two Different Formulations in Healthy Subjects: The In Vivo Effect of Particle Size. *AAPS Annual Meeting, Orlando*, 2015
- Remington's Pharmaceutical Sciences. Mack Publishing Company, 1995